# EUROPEAN PATENT SPECIFICATION

(11) **EP 3 216 485 B1**
(45) Date of publication and mention of the grant of the patent: **05.02.2020**
(21) Application number: 17000376.8
(22) Date of filing: 08.03.2017
(51) Int. Cl.: A61M 39/00, A61M 39/04, A61M 39/10, A61M 39/20, A61J 1/14

(54) **FITTING FOR A MEDICAL DEVICE SUITABLE FOR A PARENTAL OR ENTERAL INFUSION AND TRANSFUSION**
TEIL FÜR EINE MEDIZINISCHE VORRICHTUNG FÜR PARENTERALE ODER ENTERALE INFUSION UND TRANSFUSION
FERRURE POUR UN DISPOSITIF MÉDICAL APPROPRIÉ POUR UNE PERFUSION ENTÉRALE OU PARENTALE ET UNE TRANSFUSION

(30) Priority: 11.03.2016 IT UA20161560
(43) Date of publication of application: 13.09.2017
(73) Proprietor: LUC & BEL S.r.l., 41012 Carpi (MO) (IT)
(72) Inventor: Ferrari, Luca, i- 41012 Carpi (MO) (IT)
(74) Representative: Ferraiolo, Ruggero

(56) References cited:
- DE-A1- 19 728 775
- US-A- 3 633 586
- US-A1- 2011 172 592
- US-B2- 7 828 787

## Description

### Field of the invention

The invention is in the field of fittings for medical devices suitable for a parenteral or enteral infusion or transfusion and will be called "fitting/s" in the following description and claims

Such fittings are used as an intermediate member between a tube of a fluid bag and the proximal end of a cannula for infusion or transfusion to allow the fluid to pass from the bag to a patient in a sterile and controlled manner, as much as possible. The fittings substantially comprise a first part suitable for connecting with a tube of the bag and a second part suitable for engaging the cannula.

A lower room defined in the second part of these fittings is generally sterilized by using water vapor, γ or β rays or a suitable sterilizing gas, preferably ethylene oxide (Eto). Not all materials used for producing the fittings, such as PVC, can be sterilized in water vapor or with γ and β rays because the various parts of them would not react uniformly under an increase of temperature. Therefore, it's clear that the gas sterilization shall be made before closing the lower room, the gas sterilization being the only possible when the material used for producing a fitting is the preferred PVC.

### State of the art.

Fittings are known where each of said two parts is generally made by molding and then joined to the other part by bonding, although fittings made in a single piece are also known. The room defined in the second part comprises a membrane that separates the two parts and is pierceable by a cannula perforator or an infusion needle, while its lower aperture is closed by welding or by a manual application of a plug, the membrane and the plug being removable, by torsion and traction respectively, as the use of the fitting starts.

Examples of fittings of the above-mentioned two types are the following.

Document US 7,828,787 B2 discloses a fitting (20, 40) where a first part (3, 3') engages a second part intended to be connected with a bag. This first part is connected with a cannula or a needle and comprises a pierceable membrane (10, 10') which separates it from the second part. The two parts of the fitting are separate, while a plug (16) is one with the first part (3, 3'), is closed and provided with a plug tab (17, 17').

Document US 3,633,586 A discloses an end closure for a sterile tube and a syringe adapter. Basically, the sterile tube is a cannula for infusion, for intratracheal use and the like. The sterile tube is a single piece and comprises, adjacent to the needle, a soft elastomer element 46 "(plug 46") that can be punctured by a syringe needle to close again after the extraction of the needle for preventing leakage. The function of the element 46 is that of guiding a needle.

Document US 2011/172592 A1 discloses a fitting made in a single piece which defines two parts separated by a pierceable membrane made by a separate element 38 to be manually inserted in the fitting body and comprises a separate plug 60 which closes the second part of the fitting to ensure sterility.

### Drawbacks in the state of the art.

The main drawback in the state of the art is that in the most frequent cases in which the fitting is gas sterilized inside and outside, the sterilization is jeopardized in the phase in which the fitting is handled for re-closing the lower room. In the fittings where the lower room is closed by welding or a separate plug, a hermetically sealed compartment is generated which does not allow a gas sterilization.

Another drawback regarding the production technique in the case of a device made by joining two separate parts is that more molds are needed to produce the two parts of the fitting, which entails operational complication.

In the case that a fitting is produced in a single piece in which the lower aperture is closed by welding, sterilization must necessarily take place only by water vapor or γ or β rays and not by gas.

### Scopes of the invention.

The main scope of the invention is to ensure the sterilization of the fitting and this is achieved by producing a single piece comprising said two parts, a pierceable membrane separating the two parts and a closure means for the lower aperture. The closure means is connected with the fitting body by means of a flexible union tab and is indicated hereinafter by the term - aggregated movable cap - or simply - cap -.

Thus, since in the invented fitting there are not separate parts to be handled, the fitting is definitely sterile and, in the type in which the cap is provided with a suitable filtering membrane (the order of magnitude of pores in this membrane is generally of diameters ranging from 1.2 µ to 3.0 µ) for carrying out a sterilization by ethylene oxide, the fitting is sterile up to the stage wherein, removed the cap, a needle or a perforator is introduced that penetrates the pierceable membrane Another scope of the pierceable membrane, in the version for an infusion, is to prevent the infusion liquid to come in contact with a needle-guide means made of a latex-free material introduced previously in the second part adjacent to the membrane. The cap, being an enbloc piece in the fitting (aggregated plug) and being connected to the main fitting body by the union tab, can be in a closed version to make a sterilization by vapor, or by γ or β rays or, in an open version, it comprises the above mentioned filtering membrane to make a gas sterilization, thus ensuring the sterility of the lower room defined in the second part until the cap along with a section of the second part are removed using a pull tab, having used any of the above types of sterilization, and without having to resort to torsion to fracture the distal part.

The tightness of the PVC cap applied to the lower aperture in the second part is secured by undercut and bonding engaging means. Considering that the materials used for producing the fitting can not be glued, the undercut engagement means secure the desired seal even in case of use of such materials.

### Background of the invention-

The invented fitting comprises as an enbloc piece a first hollow portion, generally cylindrical, suitable for connecting with a tube of a bag, a second hollow portion suitable for engaging a cannula, a pierceable membrane separating the two parts, an aggregated movable cap suitable for closing the lower aperture of the fitting to maintain the sterility in the lower room defined in the second part and, optionally, for including into an adapted housing a suitable membrane filter for a sterilization gas injected from outside.

This cap may comprise an auxiliary aperture, preferably in the form of a hole on the top, suitable for introducing a needle or a perforator, and consequently it will be indicated as open cap. A cap non comprising said hole will be indicated as closed cap.

A first version of the fitting is suited for using a perforator and a vapor or γ or β rays sterilization and comprises an aggregated movable cap closed on the top..

A second version of the fitting is suited to the use of a perforator and is provided with an open cap and a filtering membrane previously introduced into a suitable housing in the cap and suited for filtering a sterilization gas. This fitting comprises thus:
- a first hollow part suitable for engaging under a bag;
- a second hollow part in which a lower room is defined;
- an open aggregated movable cap;
- a pierceable membrane separating the two parts;
- a filtering membrane housed into the cap,
- a pull tab which allows a section of the second part together with the cap to be removed..

The pull tab is extremely flexible and an outer projecting ring created around the second part of the fitting prevents the breakage of the casing in which is contained a fittings supply. Optionally, the fitting is devoid of a pull tab.

A third version of the fitting is suitable for using a needle in case the fitting is associated with an infusion bag and is designed for a vapor or γ or β rays sterilization, bringing an aggregated movable closed cap and where the second part comprises:
- a needle-guide means in "latex-free" material positioned adjacent to the pierceable membrane;
- an element suited for locking the needle-guide means in its position and bearing a central passage to guide the needle and make easy a correct perforation of the pierceable membrane.

A fourth version of the fitting is suitable for use with a needle in the event the fitting is designed for a gas sterilization and is associated with an infusion bag where the second part comprises:
- an aggregated movable open cap where a filtering membrane is housed for filtering the gas introduced through the cap hole;
- a needle-guide means as described above,
- an element suitable for locking the needle-guide means as described above

A feature of the aggregated movable cap is to comprise undercut engagement and locking means which engage corresponding elements in the aperture of the second part to ensure a reliable closure.

In all cases, perforator or needle version, the cap together with a section of the second part can be removed by means of the pull tab.

The inner diameter of the first part of the fitting and the outer diameter of the perforators allow a tight coupling with all the perforators in which the tip is compliant with ISO 1135-4 and 8536-4 or other current regulation.

For using a fitting associated with a bag, the procedure shall start from the removal of the aggregated movable cap together with a section of the second part by means of the pull tab and, then, by insertion of the perforator or needle, depending on the version, so that inside the lower room in the second part a sterile non contaminated volume is created whatever the type of sterilization is used.

### Advantages of the invention.

The main advantage is to give assurance that the fitting is steril until, opened the casing and ripped the cap together with a section of the second part by a pull tab, a needle or a perforator is introduced into the pierceable membrane.

From the point of view of production, the latter is made simple by the fitting obtained in a single piece molded together with the aggregated movable cap,which is a features that, before use, also avoid any handling for connecting separate parts.

### Examples of embodiments.

The invention will now be described in more detail by means of examples of embodiments and with the aid of the drawing in which, for clarity, the objects appear magnified with respect to the actual conventional sizes.
- Figures 1 to 6 are sectional views and
- Figures 7 and 8 are partial views of detail elements.

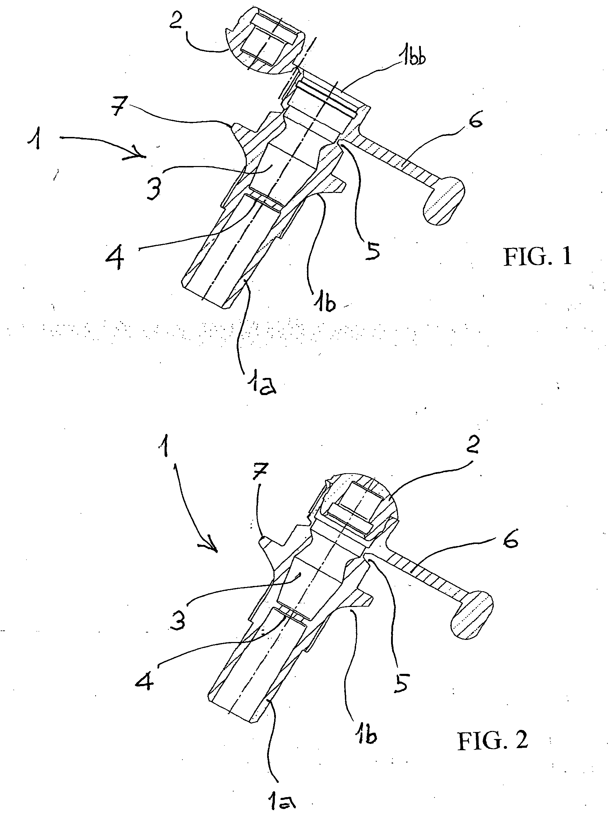

Figures 1 and 2 show a first version of the fitting 1 comprising a first part 1a, a second part 1b and an aggregated movable cap 2, closed at the top, which prepares the fitting to a vapor or γ or β rays sterilization. In Fig. 1, the cap 2 appears aggregated to the fitting via a union tab, but not engaged in the aperture 1bb of the fitting and in Fig. 2 the cap is closed on the room 3 of the second part 1b, while 4 shows the pierceable membrane in one piece with the fitting, 5 indicating the fracture zone which, by the use of the pull tab 6, allows to remove a section of the second part 1b with its cap 2, making accessible the aperture in the room 3, and 7 indicates an outer ring defined around the second part to protect the operator during use, to create an ergonomic handle, to prevent the breakage of the casing containing a quantity of fittings and, at the same time, protect the most exposed parts of the fittings supplied in bulk in a casing.

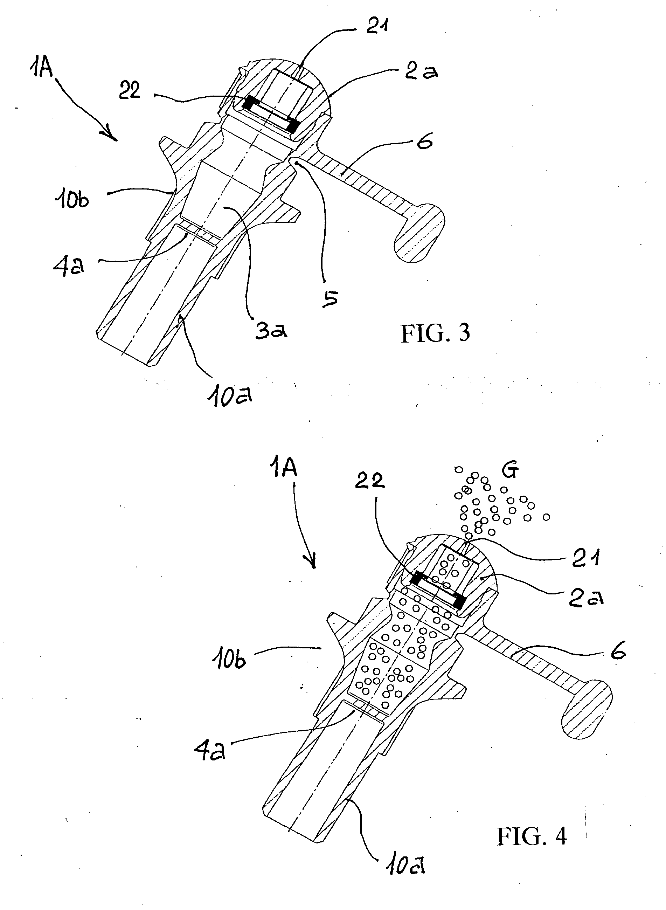

Figures. 3 and 4 show a second version of the fitting 1A in which:
- the first part 10a is adapted to engage under a bag;
- the second part 10b defines a room 3a;
- a pierceable membrane 4a separating the two parts;
- an aggregated movable cap 2a closes the aperture of the second part, a hole 21 is at the cap top and a filtering membrane 22 is housed in a suitable seat to filter the sterilization gas represented by bubbles G in Fig. 4.
- a pull tab 6, itself got together with the fitting molding, which allows the removal of the cap 2a together with a section of part 10b in correspondence of the fracture zone 5.

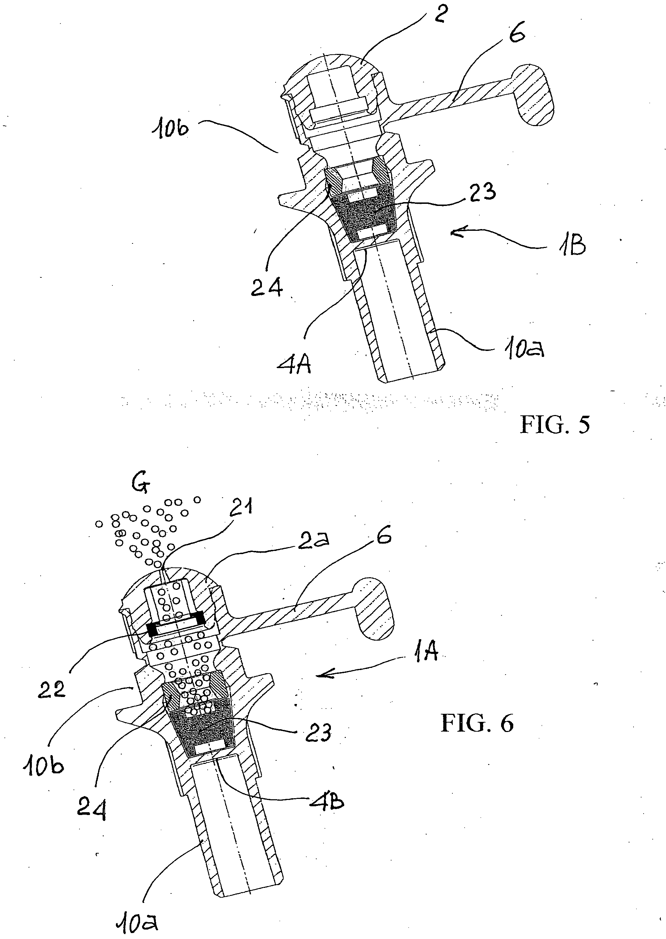

Fig. 5 shows a third version of a fitting IB adapted to a vapor or γ and β rays sterilization, comprising:
- a first part 1a adapted to be engaged under a bag;
- a second part 1b carrying an aggregated movable cap 2, closed at the top;
- needle-guide means 23 in "latex-free" material positioned adjacent to the pierceable membrane 4A that prevents the liquid present in the infusion bag from coming in contact with the latex-free material.
- a locking element 24 suitable to lock the needle-guide means 23 in its position and bearing a central passage which guides the needle so as to get a correct perforation of the pierceable membrane.
- Fig. 6 shows a fourth version of a fitting 1A suitable for use with a needle in the case of infusion bags in which the second part 10b is equipped with:
   - an aggregated movable cap 2a bearing a hole 21 at the top and a membrane filter 22 suited for filtering the sterilization gas G to be introduced through said hole;
   - a needle-guide means 23 as defined with reference to Fig. 5;
   - a locking member 24 as defined with reference to Fig. 5.

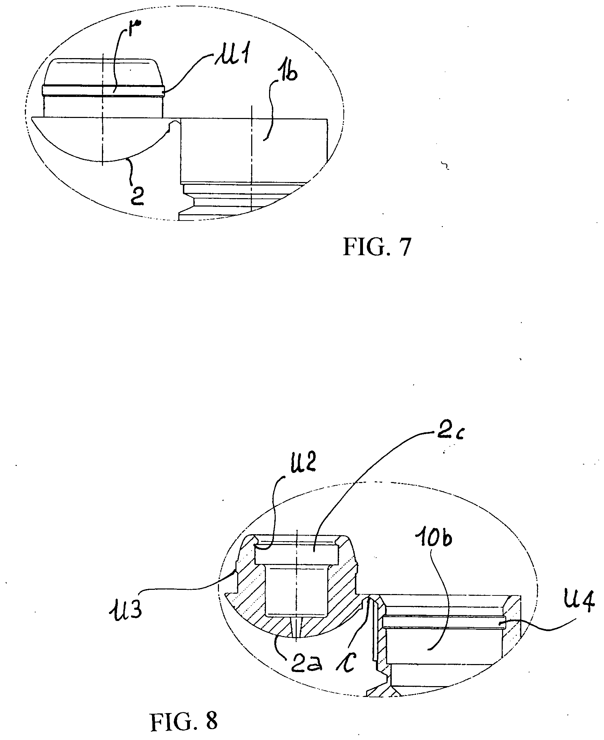

Fig. 7 shows the outer surface of a cap 2 of a fitting of which only the upper part 1b is visible. The sealing ring r comprises a tooth ring U1 which engages with a corresponding hollow ring made into the aperture of the part 1b.

Fig. 8 shows the inner and outer surfaces of an aggregated movable cap 2a and the corresponding aperture 10b to which it is connected via the union tab C obtained with the molding of the fitting. The cap has a tooth ring U2 suitable for undercut engaging with a corresponding ring made at the bottom of the aperture 10b and also has a projection U3 on the outer surface suitable for engaging in undercut with a U4 ring formed within the aperture 10b. The housing that receives a filtering membrane is indicated by 2c.

## Claims

1. Fitting (1, 1A, 1B) for a medical device suitable for a parenteral or enteral infusion and transfusion comprising a first hollow part (1a) adapted to connect with one of the tubes of a bag, a second hollow part (1b) suitable for being engaged with a cannula and a pierceable membrane (4, 4A, 4B) adapted to separate the two parts (1a, 1b) **characterized in that:**
- the first part (1a),
- the second part (1b),
- the pierceable membrane (4, 4A, 4B),
- and an aggregated movable cap (2, 2a) suitable for closing the aperture of the second part (1b) and joined to the fitting body by means of a union tab (C), are all molded together in a single piece.

2. Fitting (1) according to claim 1 **characterized in that** it comprises in one piece a pull tab (6) to remove the cap (2, 2a) and a section of the second part (1b),

3. Fitting (1) according to claims 1, 2 **characterized in that** the aggregated movable cap (2) is closed.

4. Fitting (1A) according to claims 1, 2 **characterized in that** the second part (1b) comprises:
- an aggregated movable cap (2a) provided with a hole (21);
- a filtering membrane (22) housed in a suitable seat (2c) in the aggregated movable cap (2a) and suited for filtering a sterilization gas (G) introduced through the hole (21).

5. Fitting (1A) according to claim 4 **characterized in that** the second part comprises:
- a needle-guide means (23) adjacent to the pierceable membrane (4B);
- an element (24) suitable for locking the needle-guide means and bearing a central passage for guiding the needle.

6. Fitting (1B) according to claims 1- 3 **characterized in that** the second part (1b) comprises:
- a closed aggregated movable cap (2);
- a needle-guide means (23) adjacent to the pierceable membrane (4A);
- an element (24) suited for locking the needle-guide means (23) and bearing a central passage for guiding the needle.

7. Fitting (1, 1A, 1B) according to the preceding claims, **characterized in that** an outer ring (7) made around the second part (1b) is comprised.

8. Fitting according to the preceding claims **characterized in that** the aggregated movable cap (2, 2a) comprises undercut engagement and closure element (U1, U2, U3, U4) to ensure the closure of the second part (1b).

## Patentansprüche

1. Anschlussstück (1, 1A, 1B) für eine medizinische Vorrichtung für perenterale oder enterale Infusion oder Transfusion mit einem ersten Hohlteil (1a), das ausgebildet ist, um mit einem von Rohren eines Beutels zu verbinden, einem zweiten Hohlteil (1b), das für den Eingriff mit einer Kanüle ausgebildet ist, und einer durchstechbaren Membran (4, 4A, 4B), die ausgebildet ist, um die beiden Teile (1a, 1b) zu trennen, **dadurch gekennzeichnet, dass** der erste Teil (1a),
der zweite Teil (1b),
die durchstechbare Membran (4, 4A, 4B) und
eine bewegbare Verbundkappe (2, 2a), die geeignet ist, die Öffnung des zweiten Teils (1b) zu verschließen und mit dem Anschlussstück-Körper mittels einer Verbundlasche (C) verbunden ist, zusammen als ein einzelnes Stück geformt sind.

2. Anschlussstück (1) nach Anspruch 1, **dadurch gekennzeichnet, dass** es in einem Stück eine Zuglasche (6) aufweist, um die Kappe (2, 2a) und einen Abschnitt des zweiten Teils (1b) zu entfernen.

3. Anschlussstück (1) nach Anspruch 1, 2, **dadurch gekennzeichnet, dass** die Verbundkappe (2) geschlossen ist.

4. Anschlussstück (1A) nach Anspruch 1, 2, **dadurch gekennzeichnet, dass** der zweite Teil (1b) aufweist:
eine bewegbare Verbundkappe (2a), die mit einem Loch (21) versehen ist,
eine Filtermembran (22), die in einem geeigneten Sitz (2c) in der bewegbaren Verbundkappe (2a) aufgenommen ist und zum Filtern eines Sterilisierungsgases (G) geeignet ist, das durch das Loch (21) eingebracht wird.

5. Anschlussstück (1A) nach Anspruch 4, **dadurch gekennzeichnet, dass** der zweite Teil aufweist:
eine Nadel-Führungseinrichtung (23) angrenzend an die durchstechbare Membran (4B),
ein Element (24) das zum Sperren der Nadel-Führungseinrichtung geeignet ist und einen zentralen Durchlass zum Führen der Nadel trägt.

6. Anschlussstück (1B) nach den Ansprüchen 1-3, **dadurch gekennzeichnet, dass** der zweite Teil (1b) aufweist:
eine geschlossene bewegbare Verbundkappe (2),
eine Nadel-Führungseinrichtung (23) angrenzend an die durchstechbare Membran (4A),
ein Element (24), das geeignet ist, die Nadel-Führungseinrichtung (23) zu sperren, und einen zentralen Durchlass zum Führen der Nadel trägt.

7. Anschlussstück (1, 1A, 1B) nach den vorstehenden Ansprüchen, **dadurch gekennzeichnet, dass** ein Außenring (7), der um den zweiten Teil (1b) gefertigt ist, vorgesehen ist.

8. Anschlussstück nach den vorstehenden Ansprüchen, **dadurch gekennzeichnet, dass** die bewegbare Verbundkappe (2, 2a) ein hinterschnittenes Eingriffs- und Verschlusselement (U1, U2, U3, U4) aufweist, um das Verschließen des zweiten Teils (1b) sicherzustellen.

## Revendications

1. Raccord (1, 1A, 1B) destiné à un dispositif médical adapté à une transfusion et à une perfusion parentérale ou entérale comprenant une première partie creuse (1a) susceptible d'être reliée à l'un des tubes d'une poche, une seconde partie creuse (1b) susceptible d'être mise en prise avec une canule et une membrane perçable (4, 4A, 4B) susceptible de séparer les deux parties (1a, 1b),
**caractérisé en ce que**
- la première partie (1a),
- la seconde partie (1b),
- la membrane perçable (4, 4A, 4B), et
un capuchon mobile combiné (2, 2a) susceptible de fermer l'ouverture de la seconde partie (1b) et lié au corps du raccord au moyen d'une languette de liaison (C) sont moulés ensemble en une pièce unique.

2. Raccord (1) conforme à la revendication 1,
**caractérisé en ce qu'**
il comporte en une seule pièce, une tirette (6) permettant d'enlever le capuchon (2, 2a) et un segment de la seconde partie (1b).

3. Raccord (1) conforme à la revendication 1 ou 2,
**caractérisé en ce que**
le capuchon mobile combiné (2) est fermé.

4. Raccord (1A) conforme à la revendication 1 ou 2,
**caractérisé en ce que**
la seconde partie (1b) comporte :
- un capuchon mobile combiné (2a) équipé d'un perçage (21),
- une membrane de filtration (22) logée dans un siège adapté (2c) du capuchon mobile combiné (2a) et permettant de filtrer un gaz de stérilisation (G) introduit au travers du perçage (21).

5. Raccord (1A) conforme à la revendication 4,
**caractérisé en ce que**
la seconde partie comporte :
- des moyens de guidage d'une aiguille (23) adjacents à la membrane perçable (4B),
- un élément (24) susceptible de verrouiller les moyens de guidage de l'aiguille et ayant un passage central permettant de guider l'aiguille.

6. Raccord (1B) conforme à l'une des revendications 1 à 3,
**caractérisé en ce que**
la seconde partie (1b) comporte :
- un capuchon mobile combiné fermé (2),
- des moyens de guidage d'une aiguille (23) adjacents à la membrane perçable (4A),
- un élément (24) susceptible de verrouiller les moyens de guidage de l'aiguille (23) et ayant un passage central permettant de guider l'aiguille.

7. Raccord (1, 1A, 1B) conforme aux revendications précédentes,
**caractérisé en ce qu'**
il comporte une bague externe (7) entourant la seconde partie (1b).

8. Raccord conforme aux revendications précédentes,
**caractérisé en ce que**
le capuchon mobile combiné (2, 2a) comporte un élément de mise en prise et de fermeture à contredépouille (U1, U2, U3, U4) permettant de garantir la fermeture de la seconde partie (1b).
